(19) **Europäisches Patentamt / European Patent Office / Office européen des brevets**

(11) **EP 4 670 710 A1**

(12) **EUROPEAN PATENT APPLICATION**
published in accordance with Art. 153(4) EPC

(43) Date of publication:
**31.12.2025 Bulletin 2026/01**

(21) Application number: **24791655.4**

(22) Date of filing: **03.01.2024**

(51) International Patent Classification (IPC):
**A61K 9/08** *(2006.01)*     **A61K 38/18** *(2006.01)*
**A61K 47/18** *(2017.01)*     **A61K 47/26** *(2006.01)*
**A61K 47/36** *(2006.01)*     **A61P 27/02** *(2006.01)*

(52) Cooperative Patent Classification (CPC):
Y02A 50/30

(86) International application number:
**PCT/CN2024/070255**

(87) International publication number:
**WO 2024/217067 (24.10.2024 Gazette 2024/43)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority:   **21.04.2023   CN 202310431075**

(71) Applicant: **Shanghai Tenry Pharmaceutical Co., Ltd.**
**Fengxian District, Shanghai 201400 (CN)**

(72) Inventors:
  • **FU, Dongjun**
    **Shanghai 201400 (CN)**
  • **ZHU, Guoxing**
    **Shanghai 201400 (CN)**
  • **TANG, Fang**
    **Shanghai 201400 (CN)**
  • **HE, Jiaxin**
    **Shanghai 201400 (CN)**
  • **WANG, Kai**
    **Shanghai 201400 (CN)**
  • **LI, Zhengwu**
    **Shanghai 201400 (CN)**
  • **GUAN, Lufan**
    **Shanghai 201400 (CN)**

(74) Representative: **ABG Intellectual Property Law, S.L.**
    **Avenida de Burgos, 16D**
    **Edificio Euromor**
    **28036 Madrid (ES)**

(54) **ACIDIC FIBROBLAST GROWTH FACTOR EYE DROP PREPARATION**

(57)    The present invention relates to a recombinant human acidic fibroblast growth factor preparation, and particularly relates to an acidic fibroblast growth factor eye drop preparation. In the eye drop preparation, a combination selected from histidine, Tween 80 and heparin sodium is used as a protein protective agent, without using human albumin as the protein protective agent, thereby reducing the costs, effectively improving the stability of the product, and maintaining high bioactivity for 9 months under the conditions of (25±2)°C and relative humidity (60±5)%.

**EP 4 670 710 A1**

## Description

### Technical Field

The present invention relates to the field of biomedicine, and particularly to a stable acidic fibroblast growth factor preparation.

### Background Art

Fibroblast growth factors (FGFs) are multifunctional and powerful cytokines that play an important role in promoting fibroblast metabolism and collagen formation. Fibroblast growth factors can promote the growth and reproduction of skin tissue. They regulate the division, reproduction, and growth differentiation of skin epithelial, endothelial and stromal cells, promote cell metabolism and enhance oxidation by binding to specific receptors on the cell surface. They can promote the rapid growth and reproduction of cells related to skin damage, and regulate the synthesis, secretion, and decomposition of the intercellular matrix. They can promote the regeneration of stratum corneum cells, accelerate the repair of the stratum corneum and matrix layer of the skin, and promote the growth of human skin cells. They can enhance the synthesis of protein and cell metabolism of skin cells, and have the effects of delaying skin cell aging and promoting the repair and growth of epidermal cells.

A fibroblast growth factor eye drop has an effect of promoting the repair and regeneration of cells derived from the mesoderm and ectoderm. The results of animal experiments show that the present product promotes the regeneration of corneal epithelium and the repair of corneal stroma and endothelium in rabbits with alkali burns; commercially available products include acidic fibroblast growth factor (aFGF or FGF-1) lyophilized powder and alkaline fibroblast growth factor (bFGF or FGF-2) eye drop. aFGF lyophilized powder needs to be stored and transported at 2-8°C in a light-protected environment, and it has a shelf life of about 24 months. When used, the solvent provided in the package is poured into the bottle containing rhaFGF lyophilized powder. bFGF eye drop may be used directly, but must be stored at 2-8°C and it has a shelf life of approximately 24 months. Once placed at room temperature, it should be used as soon as possible. Therefore, it is of great significance to study the stabilizer suitable for aFGF, obtain a stable aFGF pharmaceutical composition, and improve the compliance and convenience of patients; there is an urgent need in the art to provide a liquid preparation that can maintain the long-term stability of acidic fibroblast growth factor.

### Summary of the Invention

In view of the deficiencies in the prior art, the present invention provides a stable acidic fibroblast growth factor preparation and preparation method and application thereof. The components of the preparation are studied, in particular, different combinations of stabilizers are selected and the proportioning of each stabilizer is compared. A stable preparation containing acidic fibroblast growth factor is obtained through repeated experimental studies.

The present invention provides a human acidic fibroblast growth factor eye drop, which comprises a protein protective agent, a humectant, a buffer salt, and an osmotic pressure regulator, wherein the protein protective agent is selected from a combination of histidine, Tween 80, and heparin sodium.

The present invention provides a recombinant human acidic fibroblast growth factor eye drop, wherein each 100 ml of the eye drop contains 0.01-0.2 g of histidine, 0.01-0.2 g of Tween 80 and 0.005-0.1 g of heparin sodium; preferably contains 0.1-0.2 g of histidine, 0.03-0.1 g of Tween 80 and 0.01-0.05 g of heparin sodium.

The present invention provides a recombinant human acidic fibroblast growth factor eye drop, wherein each 100 ml of the eye drop contains 1 to 3 mg of the human acidic fibroblast growth factor, preferably contains 2 mg of the human acidic fibroblast growth factor.

The present invention provides a recombinant human acidic fibroblast growth factor eye drop, wherein the humectant is selected from one or more of sodium hyaluronate, chondroitin sulfate, polyvinyl alcohol, polyethylene glycol, glycerol, hypromellose, dextran, and carboxymethyl cellulose, and each 100 ml of the eye drop contains 0.05 to 1.5 g of the humectant, and preferably, each 100 ml of the eye drop contains 0.1 g of the humectant.

The present invention provides a recombinant human acidic fibroblast growth factor eye drop, wherein the buffer salt is selected from the group consisting of phosphate buffer system, histidine salt buffer system, acetate buffer system, citrate buffer system, Tris buffer system, and HEPES buffer system, and the preferred pH value of the buffer salt is 6.0 to 7.0, the concentration is 0.01 to 0.05 mol/L, and the preferred concentration is 0.02 mol/L.

The present invention provides a recombinant human acidic fibroblast growth factor eye drop, wherein the osmotic pressure regulator is selected from one or more of sodium chloride, boric acid and glucose, and preferably, each 100 ml of the eye drop contains 0.5 to 0.9 g of the osmotic pressure regulator.

The present invention provides a recombinant human acidic fibroblast growth factor eye drop, wherein the eye drop further contains an antibacterial agent.

The present invention provides a recombinant human acidic fibroblast growth factor eye drop, wherein the antibacterial agent is selected from one or more of ethylparaben, benzalkonium chloride, benzalkonium bromide, polyquaternium salt, phenylmercuric nitrate, thimerosal, benzalkonium chloride, trichlorotert butanol, hydroxyphenyl esters, and sorbitol, and preferably, each 100 ml of the eye drop contains 0.01 to 0.1 g of the antibacterial agent.

The present invention provides a recombinant human acidic fibroblast growth factor eye drop, which further comprises an antioxidant, and the antioxidant is selected from one or more of sodium thiosulfate, sodium sulfite, vitamin E, sodium ascorbate, and methionine, and preferably, each 100 ml of the eye drop contains 0.01 to 0.1 g of the antioxidant.

The present invention provides a recombinant human acidic fibroblast growth factor eye drop, which further contains a thickener; preferably, the thickener is selected from one or more of polyvinyl alcohol, carboxymethyl cellulose, methyl cellulose, sodium carboxymethyl cellulose, sodium polyacrylate, and polyvinyl pyrrolidone; and preferably, each 100 ml of the eye drop contains 0.5 to 2 g of the thickener.

The present invention has the following beneficial effects:

The eye drop of the present invention uses a combination of histidine, Tween 80 and heparin sodium as a protein protective agent, which effectively improves the stability of the product and still maintains a high biological activity for 9 months under the conditions of $(25\pm2)°C$ and relative humidity $(60\pm5)\%$.

The combined protein protective agent used in the present invention does not need to use expensive human albumin, and effectively reduces the production costs while ensuring product quality.

Through screening, the present inventors have found a variety of preferred combinations of antibacterial agents and antioxidants, which facilitate in synergistically increasing the stability of the product.

Detailed Description of Examples

In order to facilitate a better understanding of the present invention, the following examples are used to illustrate the present invention. These examples belong to the protection scope of the present invention, but do not intend to limit the protection scope of the present invention.

Example 1: The present example provides a recombinant human acidic fibroblast growth factor preparation I, the formulation and preparation method of which are as follows:

| | |
|---|---|
| Human acidic fibroblast growth factor | 2 mg |
| Sodium hyaluronate | 0.1 g |
| Histidine | 0.15 g |
| Tween 80 | 0.05 g |
| Heparin sodium | 0.02 g |
| Sodium chloride | 0.45 g |
| Trichlorotert butanol | 0.05 g |
| Methionine | 0.05 g |
| Polyvinyl alcohol | 1 g |

The preparation of 100 ml of the eye drop: in accordance with the above-described proportions, place the humectant (sodium hyaluronate), protein protective agent (histidine, Tween 80, and heparin sodium), thickener (polyvinyl alcohol), preservative (trichlorotert butanol), and antioxidant (methionine) into 80 ml of phosphate buffer (0.02 mol/L); add 0.9 g of sodium chloride, and following complete dissolution, add the human acidic fibroblast growth factor. After thorough mixing, adjust the volume to the required volume with phosphate buffer (pH 6.5, 0.02 mol/L), sterilize the solution by filtration through a 0.22 μm microporous membrane, and dispense the filtrate into plastic eye drop bottles to prepare the finished eye drops of the present invention.

Example 2: The preparation method of the present example is the same as that of Example 1, and the specific formulation is as follows:

| | |
|---|---|
| Human acidic fibroblast growth factor | 2 mg |
| Sodium hyaluronate | 0.1 g |
| Histidine | 0.15 g |
| Tween 80 | 0.05 g |
| Heparin sodium | 0.05 g |
| Sodium chloride | 0.45 g |
| Trichlorotert butanol | 0.05 g |

(continued)

| | |
|---|---|
| sodium thiosulfate | 0.05 g |
| Polyvinyl alcohol | 1 g |

**Example 3:** The preparation method of the present example is the same as that of Example 1, and the specific formulation is as follows:

| | |
|---|---|
| Human acidic fibroblast growth factor | 2 mg |
| Sodium hyaluronate | 0.1 g |
| Histidine | 0.15 g |
| Tween 80 | 0.05 g |
| Heparin sodium | 0.05 g |
| Sodium chloride | 0.45 g |
| Methyl p-Hydroxybenzoate | 0.05 g |
| Methionine | 0.05 g |
| Polyvinyl alcohol | 1 g |

**Example 4:** The preparation method of the present example is the same as that of Example 1, and the specific formulation is as follows:

| | |
|---|---|
| Human acidic fibroblast growth factor | 2 mg |
| Sodium hyaluronate | 0.1 g |
| Histidine | 0.15 g |
| Tween 80 | 0.05 g |
| Heparin sodium | 0.05 g |
| Sodium chloride | 0.45 g |
| Sodium benzoate | 0.05 g |
| Methionine | 0.05 g |
| Polyvinyl alcohol | 1 g |

**Comparative Example 1:** The preparation method of the present comparative example refers to Example 1, and the specific formulation is as follows:

| | |
|---|---|
| Human acidic fibroblast growth factor | 2 mg |
| Sodium hyaluronate | 0.1 g |
| Histidine | 0.15 g |
| Tween 80 | 0.05 g |
| Heparin sodium | 0.05 g |
| Sodium chloride | 0.45 g |
| Trichlorotert butanol | 0.05 g |
| Methionine | 0.05 g |
| Polyvinyl alcohol | 1 g |

**Comparative Example 2:** The preparation method of the present comparative example refers to Example 1, and the specific formulation is as follows:

| | |
|---|---|
| Human acidic fibroblast growth factor | 2 mg |
| Sodium hyaluronate | 0.1 g |
| Histidine | 0.15 g |
| Heparin sodium | 0.05 g |
| Sodium chloride | 0.45 g |

(continued)

| | |
|---|---|
| Trichlorotert butanol | 0.05 g |
| Methionine | 0.05 g |
| Polyvinyl alcohol | 1 g |

**Comparative Example 3:** The preparation method of the present comparative example refers to Example 1, and the specific formulation is as follows:

| | |
|---|---|
| Human acidic fibroblast growth factor | 2 mg |
| Sodium hyaluronate | 0.1 g |
| Histidine | 0.15 g |
| Tween 80 | 0.05 g |
| Sodium chloride | 0.45 g |
| Trichlorotert butanol | 0.05 g |
| Methionine | 0.05 g |
| Polyvinyl alcohol | 1 g |

**Comparative Example 4:** The preparation method of the present comparative example refers to Example 1, and the specific formulation is as follows:

| | |
|---|---|
| Human acidic fibroblast growth factor | 2 mg |
| Sodium hyaluronate | 0.1 g |
| Heparin sodium | 0.05 g |
| Human albumin | 0.1 g |
| Sodium chloride | 0.45 g |
| Methionine | 0.05 g |
| Polyvinyl alcohol | 1 g |

**Comparative Example 5:** The preparation method of the present comparative example refers to Example 1, and the specific formulation is as follows:

| | |
|---|---|
| Human acidic fibroblast growth factor | 2 mg |
| Sodium hyaluronate | 0.1 g |
| Histidine | 0.15 g |
| Human albumin | 0.1 g |
| Sodium chloride | 0.45 g |
| Trichlorotert butanol | 0.05 g |
| Polyvinyl alcohol | 1 g |

**Effect Example:** Accelerated stability test

The eye drops prepared in Examples 1-4 and Comparative Examples 1-4 were placed under conditions of $(25 \pm 2)$ °C and relative humidity $(60 \pm 5)$% for 9 months, and then an activity test was performed. The activity test method was as follows:

(1) Preparation of standard solution: take the recombinant human acidic fibroblast growth factor standard, reconstitute it according to the instructions under sterile conditions, and dilute it to contain 40 IU per 1 ml. In a 96-well cell culture plate, perform a 4-fold serial dilution, with 8 dilution levels, and 2 wells for each dilution level.

(2) Preparation of test sample solution: dilution method and treatment method are the same as those of standard samples.

(3) The above samples were immediately subjected to the test in step (4) below.

(4) Activity assay: Balb/c 3T3 cell line was cultured in complete culture medium at 37 °C and 5% $CO_2$, and the cell concentration was controlled at $1.0\times10^5$ to $5.0\times10^5$ cells/ml. The cells were used for biological activity assay 24 to 36 hours after subculture. The culture medium in the culture flask was discarded, the cells were digested and collected, and a cell suspension of $5.0\times10^4$ to $1.3\times10^5$ cells/ml was prepared with complete culture medium, and inoculated into a 96-well cell culture plate with 100 μL per well. Incubation was performed at 37 °C and 5% $CO_2$ for 24 h. Then, the culture medium was replaced with maintenance medium and cultured at 37 °C and 5% $CO_2$ for 28 to 32 hours. The maintenance solution was discarded from the prepared cell culture plate, and the standard solution and the test sample were added with 100 μL per well. Incubation was performed at 37 °C and 5% $CO_2$ for 64 to 72 hours. At the end of the culture, 20 μL of MTT solution was added to each well and cultured at 37 °C and 5% $CO_2$ for 5 to 6 hours. 100 μL of lysis solution was added to each well of the cell culture plate, which was mixed well and put into the microplate reader, and 630 nm was used as the reference wavelength. The absorbance at a wavelength of 570 nm was measured, and the measurement results were recorded.

Result calculation: The test data were processed by computer program or Linear regression calculation method, and the results were calculated as follows:

$$\text{Biological activity of the test sample (IU/ml)} = Pr \times Ds \times Es / (Dr \times Er)$$

Relative activity (%) = biological activity of the test sample (after accelerated test)/ biological activity of the test sample (at the stability baseline)

Where Pr is the biological activity of the standard, in IU/ml; Ds is the pre-dilution factor of the test sample; Dr is the pre-dilution factor of the standard; Es is the dilution factor of the test sample equivalent to the half-effective dose of the standard; Er is the dilution factor of the half-effective dose of the standard; the relative activity (%) test data after the accelerated test is shown in Table 1:

Table 1

| | Example 1 | Example 2 | Example 3 | Example 4 | Comparative Example 1 | Comparative Example 2 | Comparative Example 3 | Comparative Example 4 | Comparative Example 5 |
|---|---|---|---|---|---|---|---|---|---|
| relative activity (%) | 92.5 | 98.3 | 93.1 | 89.3 | 58.6 | 53.7 | 46.1 | 61.1 | 63.6 |

The above data show that the combination of histidine, Tween 80 and heparin sodium as a combination of protein protective agent is beneficial to improve the stability of human acidic fibroblast growth factor under accelerated test conditions; as for the effect of antioxidants and preservatives on stability, the combination of trichlorotert butanol and methionine in Example 1 is the best embodiment, and the effects of other preservatives and antioxidants are slightly worse.

The above contents cannot be used to determine that the specific implementation of the present invention is limited to these descriptions. Those skilled in the art to which the present invention belongs can make several simple deductions or substitutions without departing from the concept of the present invention, which should be regarded as belonging to the protection scope of the patent of the present invention determined by the submitted claims.

**Claims**

1. A recombinant human acidic fibroblast growth factor eye drop, the eye drop comprising a protein protective agent, a humectant, a buffer salt, and an osmotic pressure regulator, **characterized in that** the protein protective agent is selected from the combination of histidine, Tween 80, and heparin sodium.

2. The recombinant human acidic fibroblast growth factor eye drop according to claim 1, **characterized in that** each 100 ml of the eye drop contains 0.01-0.2 g of histidine, 0.01-0.2 g of Tween 80 and 0.005-0.1 g of heparin sodium; preferably contains 0.1-0.2 g of histidine, 0.03-0.1 g of Tween 80 and 0.01-0.05 g of heparin sodium.

3. The recombinant human acidic fibroblast growth factor eye drop according to claim 1, **characterized in that** each 100 ml of the eye drop contains 1 to 3 mg of a human acidic fibroblast growth factor, preferably contains 2 mg of the human acidic fibroblast growth factor.

4. The recombinant human acidic fibroblast growth factor eye drop according to claim 1, **characterized in that** the humectant is selected from one or more of sodium hyaluronate, chondroitin sulfate, polyvinyl alcohol, polyethylene glycol, glycerol, hypromellose, dextran, and carboxymethyl cellulose, and each 100 ml of the eye drop contains 0.01 to 1.5 g of the humectant, preferably each 100 ml of the eye drop contains 0.1 g of the humectant.

5. The recombinant human acidic fibroblast growth factor eye drop according to claim 1, **characterized in that** the buffer salt is selected from the group consisting of phosphate buffer system, histidine salt buffer system, acetate buffer system, citrate buffer system, Tris buffer system, and HEPES buffer system, and the preferred pH value of the buffer salt is 6.0 to 7.0, the concentration is 0.01 to 0.05 mol/L, and the preferred concentration is 0.02 mol/L.

6. The recombinant human acidic fibroblast growth factor eye drop according to claim 1, **characterized in that** the osmotic pressure regulator is selected from one or more of sodium chloride, boric acid, and glucose, and preferably, each 100 ml of the eye drop contains 0.3 to 0.9 g of the osmotic pressure regulator.

7. The recombinant human acidic fibroblast growth factor eye drop according to claim 1, **characterized in that** the eye drop further contains an antibacterial agent.

8. The recombinant human acidic fibroblast growth factor eye drop according to claim 7, **characterized in that** the antibacterial agent is selected from one or more of ethylparaben, benzalkonium chloride, benzalkonium bromide, polyquaternium salt, phenylmercuric nitrate, thimerosal, benzalkonium chloride, trichlorotert butanol, hydroxyphenyl esters, and sorbitol, and preferably, each 100 ml of the eye drop contains 0.01 to 0.1 g of the antibacterial agent.

9. The recombinant human acidic fibroblast growth factor eye drop according to claim 1, **characterized in that** the eye drop further contains an antioxidant.

10. The recombinant human acidic fibroblast growth factor eye drop according to claim 9, **characterized in that** the antioxidant is selected from one or more of sodium thiosulfate, sodium sulfite, vitamin E, sodium ascorbate, and methionine, and preferably, each 100 ml of the eye drop contains 0.01 to 0.1 g of the antioxidant.

11. The recombinant human acidic fibroblast growth factor eye drop according to claim 1, **characterized in that** the eye drop further contains a thickener; preferably, the thickener is selected from one or more of polyvinyl alcohol, carboxymethyl cellulose, methyl cellulose, sodium carboxymethyl cellulose, sodium polyacrylate, and polyvinyl pyrrolidone; and preferably, each 100 ml of the eye drop contains 0.5 to 2 g of the thickener.

## INTERNATIONAL SEARCH REPORT

| International application No. |
| --- |
| **PCT/CN2024/070255** |

| A. | CLASSIFICATION OF SUBJECT MATTER |
| --- | --- |

A61K 9/08(2006.01)i; A61K 38/18(2006.01)i; A61K 47/18(2017.01)i; A61K 47/26(2006.01)i; A61K 47/36(2006.01)i; A61P 27/02(2006.01)i

According to International Patent Classification (IPC) or to both national classification and IPC

| B. | FIELDS SEARCHED |
| --- | --- |

Minimum documentation searched (classification system followed by classification symbols)

IPC:A61K31,A61P

Documentation searched other than minimum documentation to the extent that such documents are included in the fields searched

Electronic data base consulted during the international search (name of data base and, where practicable, search terms used)

CNABS, CNKI, DWPI, VEN, CNTXT, ENTXT, ISI Web of Science, CNKI, 万方, WANFANG: 上海腾瑞制药股份有限公司, 酸性成纤维细胞生长因子, aFGF, FGF-1, rhaFGF, acidic fibroblast growth factor?, 吐温80, 聚山梨酯80, 聚氧乙烯失水山梨醇脂肪酸酯80, tween?, PS80, PS-80, 肝素钠, heparin sodium?, 组氨酸, histidine?, *氨酸, 滴眼液, 眼用, 眼药, eye drop?

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| PX | CN 116392441 A (SHANGHAI TENRY PHARMACEUTICAL CO., LTD.) 07 July 2023 (2023-07-07) <br> description, paragraphs 5-18 | 1-11 |
| Y | CN 110063936 A (ANHUI XINHUAKUN BIO-ENGINEERING CO., LTD.) 30 July 2019 (2019-07-30) <br> description, embodiment 3, and paragraph 7 | 1-11 |
| Y | CN 103720642 A (BEIJING TIDE PHARMACEUTICAL CO., LTD.) 16 April 2014 (2014-04-16) <br> claims 1-6 | 1-11 |
| Y | WO 2021263134 A1 (TREFOIL THERAPEUTICS, INC.) 30 December 2021 (2021-12-30) <br> description, paragraphs 6, 36, 238-239, 248, 358-359 and 369-371 | 1-11 |
| A | CN 101143212 A (BIOPHARMACEUTICAL RESEARCH AND DEVELOPMENT CENTER OF JINAN UNIVERSITY) 19 March 2008 (2008-03-19) <br> claims 1-4 | 1-11 |

☑ Further documents are listed in the continuation of Box C.  ☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
| --- | --- |
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **01 March 2024** | **08 March 2024** |

| Name and mailing address of the ISA/CN | Authorized officer |
| --- | --- |
| **China National Intellectual Property Administration (ISA/ CN)** <br> **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
| --- |
| **PCT/CN2024/070255** |

| C. | DOCUMENTS CONSIDERED TO BE RELEVANT | |
| --- | --- | --- |
| Category* | Citation of document, with indication, where appropriate, of the relevant passages | Relevant to claim No. |
| A | CN 109260147 A (ZHUHAI ESSEX BIO-PHARMACEUTICAL CO., LTD.) 25 January 2019 (2019-01-25)<br>      claims 1-7 | 1-11 |
| A | CN 113274486 A (SHANGHAI TENRY PHARMACEUTICAL CO., LTD.) 20 August 2021 (2021-08-20)<br>      claims 1-6 | 1-11 |
| A | CN 114010763 A (SHANGHAI TENRY PHARMACEUTICAL CO., LTD.) 08 February 2022 (2022-02-08)<br>      claims 1-10 | 1-11 |
| A | US 2020157164 A1 (ZHUHAI ESSEX BIO-PHARMACEUTICAL CO., LTD.) 21 May 2020 (2020-05-21)<br>      claims 4-15 | 1-11 |
| A | EP 0408146 A2 (MERCK & CO., INC.) 16 January 1991 (1991-01-16)<br>      claim 8, and description, page 4, lines 31-44 | 1-11 |
| A | 冯秀萍等 (FENG, Xiuping et al.). "重组人酸性成纤维细胞生长因子滴眼液的制备及其稳定性 (Preparation of Recombinant Human Acid Fibroblast Growth Factor Eye Drops)"<br>中国生物制品学杂志 (*Chinese Journal of Biologicals*).<br>Vol. 21, No. (10), 31 October 2008 (2008-10-31), 884-886<br>      pp. 884-886 | 1-11 |

## INTERNATIONAL SEARCH REPORT
### Information on patent family members

| International application No. |
| --- |
| **PCT/CN2024/070255** |

| Patent document cited in search report | | | Publication date (day/month/year) | Patent family member(s) | | | Publication date (day/month/year) |
| --- | --- | --- | --- | --- | --- | --- | --- |
| CN | 116392441 | A | 07 July 2023 | None | | | |
| CN | 110063936 | A | 30 July 2019 | None | | | |
| CN | 103720642 | A | 16 April 2014 | None | | | |
| WO | 2021263134 | A1 | 30 December 2021 | None | | | |
| CN | 101143212 | A | 19 March 2008 | None | | | |
| CN | 109260147 | A | 25 January 2019 | None | | | |
| CN | 113274486 | A | 20 August 2021 | None | | | |
| CN | 114010763 | A | 08 February 2022 | None | | | |
| US | 2020157164 | A1 | 21 May 2020 | KR | 20200021521 | A | 28 February 2020 |
| | | | | KR | 102480402 | B1 | 22 December 2022 |
| | | | | US | 11254723 | B2 | 22 February 2022 |
| | | | | JP | 2020533015 | A | 19 November 2020 |
| | | | | JP | 7044870 | B2 | 30 March 2022 |
| | | | | SG | 11201912799 | SA | 30 January 2020 |
| | | | | MY | 194351 | A | 29 November 2022 |
| | | | | WO | 2018232750 | A1 | 27 December 2018 |
| | | | | EP | 3643784 | A1 | 29 April 2020 |
| | | | | EP | 3643784 | A4 | 05 May 2021 |
| EP | 0408146 | A2 | 16 January 1991 | CA | 2020720 | A1 | 14 January 1991 |
| | | | | JPH | 03175994 | A | 31 July 1991 |
| | | | | EP | 0408146 | A3 | 12 June 1991 |

Form PCT/ISA/210 (patent family annex) (July 2022)